# EUROPEAN PATENT APPLICATION

(11) **EP 1 593 738 A1**
(43) Date of publication of application: **09.11.2005**
(21) Application number: 04706596.6
(22) Date of filing: 30.01.2004
(51) Int. Cl.: C12N 7/04, A61K 39/12, C12N 15/86, C12N 15/40

(54) **VIRUS-LIKE PARTICLE VACCINE CONTAINING DENGUE VIRUS RECOMBINANT REPLICON AS ITS CARRIER**

(30) Priority: 30.01.2003 CN 03115273; 30.01.2003 CN 03115272
(71) Applicant: Shanghai Tengen Biomedical Co. Ltd., Shanghai 200003 (CN); Tengen Biomedical Co., Rockville, MD 20850 (US); Beijing Oriental Tengen Technology Development Co. Ltd., Beijing 100006 (CN)
(72) Inventor: PANG, Xiaowu, Rockville, MD 20850 (US)
(74) Representative: Bohmann, Armin K.
(86) International application number: PCT/CN2004/000088
(87) International publication number: WO 2004/072274

(57) **Abstract**

The present invention relates virus-like particle vaccine containing dengue virus recombinant replicon as its core and its preparation method. Such vaccine can efficiently express antigen in the infected tells. Because dengue virus can infect dendritic cells, it can efficiently present antigen and has immunity effects. Further, different dengue virus types can be used to repeatedly produce efficient immune response, which strengthen body's immune response against the pathogen containing such antigen. Such vaccine can prevent and cure cancer and viral diseases.

## Description

### Field of the Invention

The invention relates to dengue virus (DEN) and in particular to a recombinant virus that is prepared by replacing DEN structural protein genes with non-dengue transgenes, such as, therapeutic antigens, and its production and application.

### Background

Cancer threatens human health seriously. Some cancers have been linked to a variety of viruses, most notably human papillomavirus (HPV), which is responsible for cervical cancer. The prevalence of HPV infection of the female genital tract has a positive correlation with the pathological progression of the diseased cervix presenting in the normal progression of the disease beginning with chronic cervicitis → pseudocondyloma → verruca lesions → condyloma acuminate → cervical intraepithelial neoplasia → cervical cancers. HPV infection has been shown to be extremely common affecting 10-40 percent of women in China and 40-60 percent of women in Europe and the Western Countries.

Cervical cancer is the second most common cancer after breast cancer among women worldwide. According to WHO, there are 1.7 million cervical cancer patients diagnosed worldwide, and approximately 500,000 new cases are diagnosed per year. There are as many as 200,000 deaths per year. There are nearly 140,000 cervical cancer patients in China, and 40,000 new cases are diagnosed every year. Moreover, more than 75 percent of cervical cancers were found to be associated with HPV 16 and/or HPV 18 infection.

The current treatments for cervical cancer are surgery, radiotherapy, chemotherapy, and occasionally such treatments are combined with therapies known as Chinese medicine, herb medicine, and so on. Unfortunately, the effect is not satisfactory, and the five-year survival rate is 65 percent. The prognosis following the recurrence of disease is extremely poor, with only five percent survival over two years. The expense for cervical cancer screening and therapy in the USA is approximately 570 million dollars per year.

The three traditional methods for cervical cancer therapy, surgery, chemotherapy and radiotherapy, have many limitations, such as high disease recurrence rate, serious side effects and tolerance. Because of its theoretically high specificity and low side effects, biotherapy, especially immunotherapy and gene therapy, is being viewed as a new strategy for tumor therapy. The sciences of immunotherapy and gene therapy are now being applied in the treatment and cure of many different diseases and the promise of biotherapy is being realized.

In the area of cancer treatment vaccines, a number of different strategies are being pursued.

### Inactivated vaccines:

The earliest tumor vaccines with good safety, cannot induce effective cellular immune responses because the antigen generally cannot be expressed by a host cell.

### DNA virus vector vaccines:

A kind of live vaccine with a DNA genome as the vector, can effectively replicate and express an oncogene in vivo and can continually stimulate the cellular immune system to produce cytotoxic T cells (CTLs) against tumors. But to avoid oncogenicity, only parts of the oncogene are commonly employed as the antigen. Thus the immunogenicity of the antigen is reduced. Another problem of this type of vaccine is safety.

Borysiewicz LK et al (Lancet. 1996 Jun 1:347(9014):1523-7.) reported an HPV vaccine with vaccinia virus as vector. But, most people have antibody to vaccinia so its application is limited. Moreover, boosters of the vaccine are useless because of the existing or developed immune response to the vector.

WO0153467 provides recombinant yellow fever virus (YFV), which comprise exogenous nucleotide sequences. Infection of a host cell with a recombinant YFV provides for expression of the exogenous nudeic acid in a host cell and production of an antigenic polypeptide encoded by the exogenous nucleic acid. Such recombinant YFV are useful in eliciting an immune response to the exogenous polypeptide. But the recombinant YFV, retaining the whole YFV genome without deletion of some relevant cis genes, is replicable and thus, infectious. So the recombinant YFV as a vaccine is not safe, although its preparation does not need a packaging system.

WO9928487 provides an expression and delivery method of exogenous sequences by the flavivirus, kunjin virus (KUN). KUN replicons are constructed by the deletion of part of the KUN structural genes and the insertion of an exogenous sequence (i.e., non-KUN) into the genome, followed by packaging as KUN virus-like particles (VLP) in host cells, along with infection by other virus carrying the complementing coding region of the deleted KUN structural protein gene(s). However, the titer of VLP was unsatisfactory. Also, the booster immunization will be less effective. The KUN genome is stable in plasmids, which can be cloned and recombined with traditional methods. However, that strategy does not apply to other flaviviruses. For example, DEN sequences have lower stability in plasmids than KUN, probably because KUN belongs to the Japanese encephalitis virus group whereas DEN belongs to the different Dengue virus group. The two groups have only 45% homology at the level of the genome. So it is hard to construct DEN replicons and to obtain DEN VLPs directly following the teachings of WO9928487.

WO02072803 teaches a method for construction of DEN subgenomic replicons and use thereof for a DEN vaccine. Nevertheless, it is difficult to apply the invention because there is some insufficient information in the application.

### Antigen presenting cells (APC) vaccines:

The preparation and use of APC vaccines is, for example, based on the approach whereby dendritic cells (DC) from a patient are cultured and amplified, transformed to express a tumor-associated antigen (TAA) or tumor-specific antigen (TSA), and the treated DC cells then become an APC vaccine for the patient. The APCs will activate T cells of the patient to kill tumor cell when the vaccine is injected into patient. However, such an APC vaccine is patient-specific so these vaccines can't be administered en masse to a wide population. Moreover, this therapy is time-consuming, labor-consuming, is associated with a high-cost because it is difficult to culture DCs, it is costly to amplify the transformed cells, it can be difficult to activate and obtain maturation of DCs for proper and effective antigen presentation and there are quality control issues because of the difficult vaccine preparation process and the individual nature of the product.

In summary, there is an urgent requirement for novel vaccines with high capacity, host cells are long-lived, can carry large inserts and are easy to propagate. Such vaccines also are not oncogenic and can be administered repeatedly.

### Summary of the Invention

The invention aims to provide novel products applicable to industrial production, which have good safety, high titer, good booster effects, especially the vaccines against tumor and virus.

In a first aspect the invention provides recombinant DEN with a deletion of preM and comprising of follow nucleotide sequences at least:
a) DEN 5' untranslated region (5'-UTR);
b) The coding region of the first 20 amino acids of the DEN capsid protein;
c) The coding region of the signal peptide of the DEN NS1 protein;
d) The coding region of all the DEN non-structure proteins
e) DEN 3'-end untranslation region (3'-UTR)
f) Exogenous nucleotide sequences (i.e., non-DEN) between b) and c)

In preferred embodiments, the 3' end of any exogenous nucleotide sequence contains 3' release elements upstream of the coding region of the NS1 signal peptide, which can be the 5' release elements. These release elements can be a nudeotide sequence obtained from the autoprotease of foot-and-mouth disease virus (FMDV) (SEQ ID NO: 3), or nucleotide sequence encoding the substrate of signal peptide protease (SEQ ID NO: 44) or both.

In another preferred embodiment, DEN is obtained from any one of the following: DEN type I, DEN type II, DEN type III, or DEN type IV.

In another preferred embodiment, the coding region of the NS1 signal peptide is composed of the about last 72 nucleotides of the 3' terminus of the E gene. Altematively, a suitable signal peptide encoding sequence can be subcloned upstream in an operable situs of the NS1 coding sequence.

In another preferred embodiment, the exogenous sequence encodes an HPV antigen, immunoregulators or both.

In a second aspect, the present invention also provides VLPs comprising a DEN recombinant replicon and DEN structural proteins.

In a third aspect, the present invention also provides pharmaceutical compositions containing a DEN recombinant replicon or a DEN-VLP, and pharmaceutically acceptable carrier.

In a fourth another aspect, the present invention also provides use of a DEN recombinant replicon or a DEN-VLP for prophylactic and therapeutic use as a drug for tumor and viral infection.

In a fifth aspect, the present invention also provides cells for a packaging system encapsulating DEN recombinant replicons into VLP. These packaging cells are selected from:
a) Cells transfected by a plasmid with deleted structural genes of a Dengue recombinant replicon,
b) Cells transfected by a vector derived from a helper virus containing sequences of the structural proteins which are deleted from the DEN recombinant replicon (a), and
c) Cells integrated with structural genes which are deleted from the DEN recombinant replicon (a) in the genome thereof, and above cells express DEN structural proteins for complementing replicon packaging and the structural gene expression doesn't affect the growth of the host cells.

In a sixth aspect, the present invention provides a method for production of a virus-like particle (VLP) as herein described comprising the steps of:
a) Introducing into a DEN packaging cell line, a DEN recombinant replicon or a VLP containing said replicon of claim 1;
b) Culturing that cell line either so it can express the DEN structural proteins; or introducing into that cell line replicons of a helper virus containing a DEN structural gene if the cell line cannot express that DEN structural gene, then culturing the cell line; and
c) Collecting DEN-VLP containing DEN recombinant replicons.

In another preferred embodiment, said packaging cells contain a DEN structural protein expression vector, selected from a vector with an NS3 deletion and a Tet-regulated vector.

### Detailed Description of the Drawings

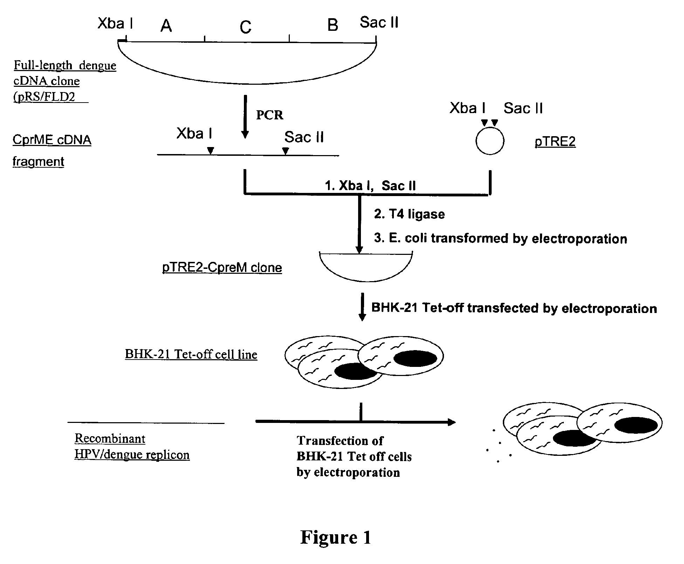

Fig 1 shows preparation of a Dengue vector carrying HPV sequences (in method 1 of example 4)

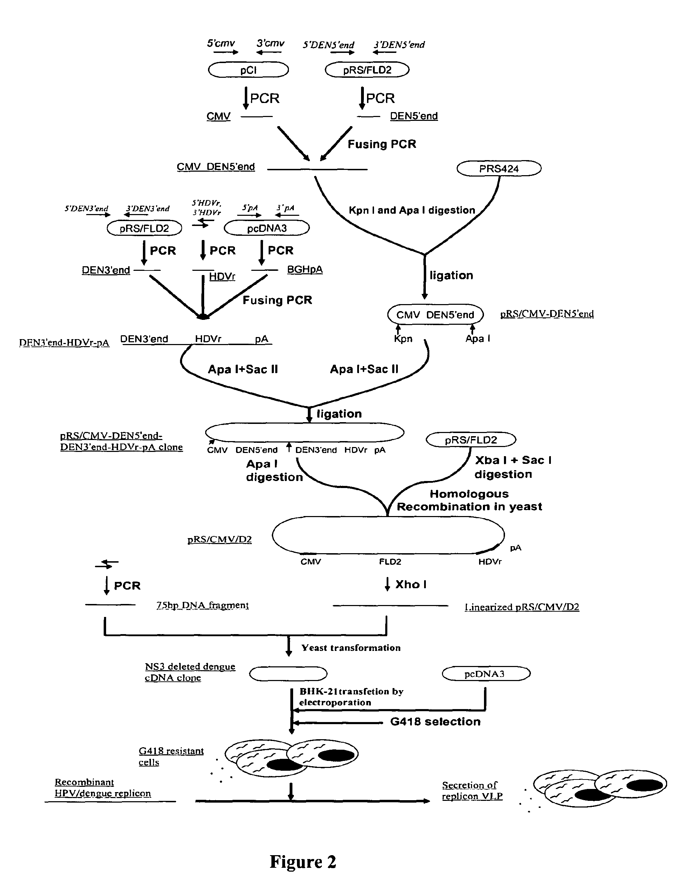

Fig 2 shows an alternative preparation of a Dengue vector carrying HPV sequences by another method (in method 4 of example 4)

### Detailed Description of Preferred Embodiments

The present invention relates to materials and methods for making a DEN-VLP preparation, as exemplified in the construction of a VLP carrying expressed HPV sequences with good safety, high titer and good booster effects.

"Nt," as used herein, means a nucleotide.
"Release element" refers to nucleotide sequences at the 3' end of a structural polypeptide coding sequence or at the 5' end of the NS1 signal peptide-coding region, used to enhance release of the polypeptide by a signal peptide protease without unrelated sequences after protein translation. The preferred release element selects from nucleotide sequence encoding Foot-And-Mouth virus hydrolytic enzyme as SEQ ID NO: 3 showed and nucleotide sequence encoding signal peptide hydrolytic enzyme substrate as SEQ ID NO:44 showed or their combination. Usually there is only one "release element", but there are many "release element" occasionally.

The terms "immunological activity" or "immunogenicity" refers to the ability to induce a specific humoral and/or cellular immunity of a mammal by natural, recombinant or synthetic immunogens, such as peptides.

The terms "antigen polypeptide" or "antigen peptide" refers to the amino acid sequence eliciting an immune response of a mammal whether single or combined with other helper molecules (for example, Human histocompatibility antigen (HLA) I or II).

The term "immune response" refers to a cellular and/or humoral immune response, for example, sufficient to inhibit or prevent, for example, infection or disease caused by microbes.

The terms "object," "individual" or "patient" refers to any object which needs diagnosis or therapy, especially mammals, such as human. Other objects include other mammals, such as, cow, dog, cat, cavy, rabbit, rat, mouse, horse etc.

Based on the experiments, the DEN recombinant replicon will have the following characteristics.
1. All of the full non-structural gene sequences must be present in the replicon. Any deletion of a structural gene or loss of expression of a structural gene must be one that does not have a negative impact on non-structural protein expression and doesn't result in a frame shift mutation of a non-structural protein and a signal peptide thereof.
2. The first 60 or so nt of the C protein gene (the coding sequence of the first 20 amino acids of the C protein, that is, the amino terminus of the capsid protein) must be present in the vector because those sequences are complementary to the 5'-UTR and 3'-UTR sequences which form a circle for DEN gene replication and virus packaging. If that region is shorter than 60 nt, the efficiency of VLP formation will decrease. Sequences longer than 60 nt (for example, 81, 99, 120, 150 and 180 nt) can form VLP, however, the possible length of any exogenous gene will decrease accordingly because of packaging limitations. So the optimal dimension is retention of about the first 60-150 nt of the 5' terminus of the C protein gene, optimally 60-120 nt.
3. The non-structural protein NS1 signal peptide, namely the last 24 or so amino acids of the E protein, must be present in the replicon. The corresponding coding sequence is the last about 72 nts of the E protein gene. If shorter than 72 nts, the efficiency of VLP formation will decrease. If longer than 72 nt (for example, 81, 99, 120 or 150 nt), the vector can still form a VLP, however, the length of the exogenous gene insert will decrease accordingly because of packaging limitations. So the optimal dimension is retention of 72-150 nt, optimally 60-120 nt.
4. The deletion of a structural gene and insertion of an exogenous gene in the deleted site can't affect NS1 signal peptide function, as well as function of the downstream non-structural genes. The DEN structural protein-coding region includes C-preM-E. Usually, portions of the C-preM-E coding sequence can be deleted (about 100-2000 bp, optimally 500-2000 bp). Another optimal method is complete preM deletion to enhance safety.

The DEN virus that can be used to make a vector in the present invention has no special limitation and can be any subtype of DEN Virus. Four subtypes of DEN genome that can be used, for example, have the following ATCC accession numbers:
DEN virus type I M87512;
DEN virus type II M29095;
DEN virus type III M93130; and
DEN virus type IV AF289029.

DEN virus has a property of being bound by dendritic cells, which can enhance vaccine effects. Furthermore, DEN has an ADE property (antibody dependent enhancement of infection), which refers to the observation that after a first immunization of DEN, antibody dependent infection is enhanced when the host is immunized with a different DEN subtype. So DEN is effective for repetitive, booster immunization. The ADE phenomena can be used for exogenous protein expression systems, which not only avoid neutralization of vector caused by repetitive immunization, but also increases the efficiency of infection of vectors into cells and exogenous protein delivery efficiency. Because DEN has four subtypes, any one can be used for an exogenous expression vector, through further inoculation with different DEN recombinant replicons, that is, of a different subtype, tends to intensify immunization efficiency.

Exogenous genes used in the present invention have no special limitation. It can be any exogenous coding sequence, such as an aptamer, siRNA, ribozyme and the like, any therapeutic gene, such as a gene encoding an antibody to VEGF, an interferon, a cytokine and so on, a tumor antigen gene, a virus antigen gene, an immunoregulator gene and so on. The representative antigens include (but not limited to): human HPV antigen (such as the E6 or E7 protein of the 16 and 18 subtype), HIV antigen, HBV antigen, HCV antigen, EBV antigen, HTLV-1 antigen, MAGE, BAGE, CAGE etc. The length of an exogenous gene has no special limitation, usually the foreign coding sequence is from about 100 bp to about 2000 bp, optimally from about 150 bp to about 1200 bp. Furthermore, a release element, such as 2A, can be added at the 3' end of an exogenous gene to ensure a high level of expression of the expressed sequence. If the intercalated exogenous gene is a tumor antigen gene or a virus antigen gene, said VLP can be used for prevention and therapy of tumor and virus diseases.

Taking human papilloma virus (HPV) as an example, the present invention provides preventative and therapeutic compositions against diseases caused by HPV infection. Therein said composition contains a VLP packaged using a DEN recombinant replicon using necessary complementing DEN structural proteins. Therein said exogenous nucleic acid sequence of the DEN recombinant replicon encodes an antigen of one or many HPV subtypes. The vector can contain also a sequence encoding an immunoregulator, or the immunoregulator sequence may be carried by a second vector. Therein said HPV antigen protein can be E6, E7 or an E6/E7 fusion or composite molecule oncoprotein of HPV 16 or HPV 18, or can be an HPV major capsid protein L1 or minor capsid protein L2. Therein said immunoregulator can be a gene sequence encoding a polypeptide with an immunoregulating activity obtained from, for example, IL2, IL 12, IL 18, GM-CSF etc. or a functional portion thereof.

The composition provided by said invention has the advantage of being relatively non-immunogenic to the host, and thus can be administered repeatedly with effect. Such an antihuman papilloma virus composition provides prevention and therapy for diseases caused by papilloma virus, such as chronic cervicitis, pseudocondyloma, verrucous lesions, condyloma acuminata, cervical intraepithelial neoplasia, cervical cancer etc.

Construction of said replicon can be processed as follows:
1. Construction of a full-length DEN genome sequence.
   This can be fulfilled by normal PCR and ligation techniques as known in the art.
2. Deletion of parts of one or more DEN structural genes.
   This can be fulfilled by homologous recombination or synthesis of a deleted sequence that is cloned into and replaces the portion to be deleted from the DEN genome. A known method is homologous recombination in yeast.

1. Insertion of an exogenous gene coding sequence.
   Though the exogenous gene can be inserted into regions with partial structural gene deletion by a known method, a preferred method is homologous recombination in yeast where sequences homologous to two sites flanking the insertion site are ligated to the two ends of the exogenous gene, introducing said construct into yeast along with a DEN genome DNA with a structural gene deletion, then obtaining the replicon carrying the exogenous gene inserted therein through homologous recombination. This recombination incidence approaches 100%, making construction relatively easy, controllable and stable.

After obtaining the replicon, it can be introduced into packaging cells to produce VLP. A normal method is after the DEN replicon is introduced into cells, and then the helper virus expressing a necessary structural protein is introduced into those cells. Another method is construction of expression packaging cells with an inducible or constitutive plasmid carrying the DEN structural gene as a vector and then introduction of the replicon (or VLP) into these cells to produce VLP. For example, constitutive packaging cells can be made that do not express a functional NS3 gene. The NS3 gene possesses a special packaging signal sequence and thus, a DEN genome with an NS3 deletion can't be packaged into a VLP. So if the packaging cell doesn't have a functional DEN NS3 gene, it can't be packaged into VLP themselves. VLP produced by this packaging cell only contains a DEN recombinant replicon, and thus, there is no need to screen amongst the replicons for those that are recombinant.

Specifically, methods include:
a) The packaging cells can contain tetracycline-regulated gene expression boxes for expressing a DEN structural protein. Recombinant DEN RNA replicon is introduced into packaging cell to produce VLP containing DEN replicon.
b) After a recombinant DEN replicon is introduced into cells and cultured (for example, about 24 hrs), a helper virus, for example, an alpha virus replicon, expressing a complementing DEN structural protein (or other expression vector) is introduced into said cells to produce VLP containing DEN replicon.
c) If the packaging cell expresses a DEN genome with partial deletion of NS3 gene expression, a recombinant DEN replicon is introduced into said packaging cell to produce a VLP containing DEN replicon.
d) After infected with a VLP and cultured, cells are infected by a helper virus, such as an alpha virus replicon, expressing a complementing DEN structural protein (or other nonreplicable virus vector) to produce a VLP containing a DEN replicon.

### Pharmaceutical Compositions

The present invention also provides various compositions comprising such a recombinant DEN replicon and/or a VLP, including a pharmaceutical composition.

Various compositions comprising such a recombinant DEN can include different buffers according to practical purposes and substances suitable to other purposes required of a pharmaceutical delivery form, as known in the art. These compositions generally contain pharmaceutically available carriers, diluents and excipients as known in the pharmaceutic arts, "Remington: Pharmacology and Pharmacological Practice" 19^{th} ed. (1995) Mack Publishing Co.

Pharmaceutical compositions can be made into a variety of forms, such as injection, solid forms, such as grains, tablets, pills, suppositories and capsules, other liquid forms, such as a suspension, a spray etc. The pharmaceutical grade organic or inorganic vectors and/or attenuants suitable to oral or local use can be used for dispensing of various compositions including therapeutically active compounds. For example, some of the known carriers include water for injection, plant and animal oil and fats, and so on, as known in the art. The stabilizer, wetting agent, emulsifier, salt suitable for controlling osmolarity, various buffers suitable for maintenance of appropriate pH, surfactants, permeants to enhance transdermal movement and so on can be used for auxiliary materials as known in the art.

The said recombinant DEN can be put into dispensing means as known in the art. Thus, for example, the VLP can be put into dispensing through familiar methods of this field and suitable pharmaceutical or transforming vehicles. The suitable vehicles are sterile saline. Other aqueous or inaqueous iso-osmotic sterile injections and sterile suspensions also can be used (pharmaceutically available vehicles, also familiar to technicians of this field).

Moreover, the said compositions can also contain other components such as an adjuvant, a stabilizer, a pH regulator, a preservative etc. These components are familiar to technicians of this field. Adjuvants include but are not limited to an aluminum adjuvant, a saponin adjuvant, a Ribi adjuvant (Ribi ImmunoChem Research Inc., Hamilton, MT), a Montanide ISA adjuvant (Seppic, Paris, France), a Hunter's TiterMax adjuvant (CytRx Corp., Norcross, GA), a Gerbu adjuvant (Gerbu Biotechnik GmbH, Gaiberg, Germany) and so on. In addition, other components for regulating and modifying the immunological response can be used.

### Administration pathway and dose

The said recombinant DEN replicon and/ or VLP can be administered to an individual through known methods. The said composition is often administered through a normal vaccine administering pathway or modeling pathogen infection pathway. The pharmaceutically available vehicles can be used when administrating vaccine compositions.

Normal and pharmaceutically available administration pathways include intranasal, intramuscular, intratracheal, subcutaneous, intracutaneous, endovaginal, intrapulmonary, intravenous, nasal, oral or other extraintestinal pathways. Combined administration can be made if needed or it can be regulated according to the transgene, the disease, the disease condition and so on. The vaccine composition can be administered as a single dose or in multiple doses, and also contain a booster dose to elicit and/or to maintain immunity.

"Effective dose" is given in an amount such that the availability of DEN recombinant replicon and/or VLP is of an amount so as to elicit an immune response and effectively prevent a host against a virus infection, tumor or other source of pathology etc. Usually, after infecting host cells, every dose of vaccine is enough to produce from about 1 to about 1000 ug, or about 1 to about 200 ug, or about 10 to about 100 ug of transgene. The vaccine effective dose calculated with recombinant DEN nucleic acids as a basis usually includes administrating about 1 to about 1000 ug nucleic acids. Furthermore, the average range of vaccine effective dose is about 10² to about 10⁹, about 10³ to about 10⁷, or about 10⁴ to about 10⁵ plaque forming units (PFU). The optimal dose of vaccine can be determined, for example, by antibody titer of experimental objects and standard investigation methods of other reactions, as known in the art. Whether a booster dose is needed can be detected by supervising immunity levels using immune assays as known in the art. After evaluating serum antibody titer, one or more booster doses can be administered. Administering adjuvant and/or immunological stimulant can enhance an immune response to the target transgene.

### Compared with known technology, the present invention has the following advantages:

1. Enhancement of immunological effectiveness.
   a) The dendritic cell is the most effective antigen presenting cell. In the present invention, antigen is effectively expressed and presented in said infected cells eliciting an effective immune response against antigen with a DEN replicon as vector, capitalizing on the affinity of DEN for dendritic cells..
   b) Utilizing the characteristic of different DEN, repetitive infection and the ADE phenomenon, the user can inoculate repetitively to enhance immunological effects.
   c) As tumor antigen and virus antigen, an E6 and/or E7 recombinant DEN replicon can express E6 and/or E7 antigen in cells for an extended time and effectively can stimulate immunologic reaction to antigen for an extended period.
2. Enhancement of vaccine safety.
   a) Recombinant DEN replicon RNAs replicate and are expressed exdusive in the cytoplasm, and thus, it is difficult for recombination to occur in the somatic cell genome.
   b) The recombinant DEN replicon is a recombinant DEN RNA with a structural gene deletion. That replicon only replicates and expresses in somatic cells and doesn't form infectious virus particles.
   c) The immune system has the ability to recognize and clear recombinant replicon. As a result, it isn't permanently present in vivo.
3. Little treatment distress.
   a) Administration just a few times, reducing patient distress in the therapy process.
   b) The composition operates quickly in the host, thereby shortening the course of therapy.
4. Low cost:
   a) The packaging cells expressing a DEN structural protein are transfected by a DEN recombinant replicon to produce VLP and then are transfected by VLP again to produce substantial quantities of VLP, increasing VLP construction efficiency. That avoids massive replicon construction, shortens vaccine production, increases vaccine production efficiency and decreases cost.
   b) The constitutive expressing packaging cells without NS3 expression are transfected by a DEN recombinant replicon to produce VLP. NS3 has a special packaging signal sequence and a DEN genome with an NS3 deletion can't be packaged into a VLP. So, packaging cells can't package and form VLP themselves. VLP produced by this packaging cell contains recombinant DEN replicon only, and there is no need to screen virus particles for recombinants, thereby enhancing construction efficiency.

The invention now is exemplified in the following representative examples. Clearly, these examples only clarify and don't limit this invention. The experimental methods taught herein, many of which are known in the art, can be performed practicing such known methods, such as those taught in Sambrook et al., Molecular Cloning: A Laboratory Manual (New York, Cold Spring Harbor Laboratory Press, 1989) or recommended by manufacturers.

### Example 1

### Construction of Full-length DEN Virus cDNA Clones

1. Full-length DEN cDNA clones were constructed by incorporation of full-length DEN cDNA into plasmid pRS424, wherein said dengue sero-type II (DEN II, NGC) (ATCC#VR-1255) virus and plasmid pRS424 (ATCC#77105) were obtained from ATCC, Manassas, VA.
   a) Three DEN cDNA fragments (3'-, 5'- and middle cDNA) were produced by PCR with DEN RNA as template. 5'-cDNA contains an Xbal restriction site and an Sp6 enhancer sequence at the 5' end. The 3'-cDNA contains a Sacl restriction site at the 3' end. The middle cDNA contains sequences identical to the 3' end of the 5'-cDNA and the 5' end of the 3'-cDNA. 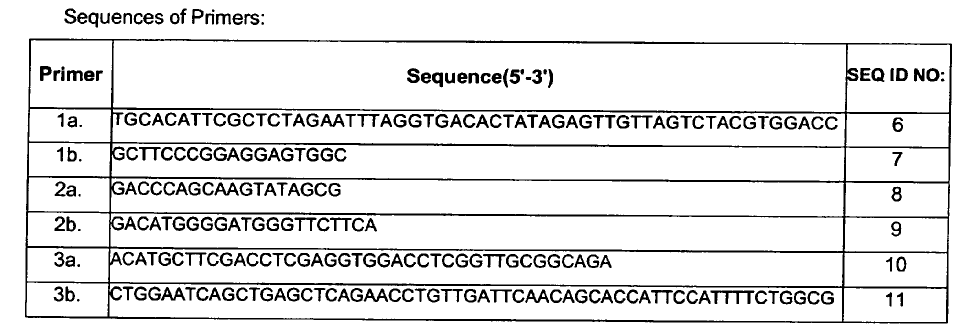 The resulting three DEN cDNA fragments are 5484 bp, 2530 bp and 2922 bp, respectively, in length.
   b) The 3' cDNA and the 5' cDNA fragments were ligated into plasmid pRS424 practicing standard methods.
   c) Using known methods of homologous recombination in yeast, the middle cDNA fragment was inserted into the said clone to produce a full-length DEN cDNA clone, pRS/FLD2.
2. Construction of DEN replicon cDNA clones with structural protein gene sequences deleted
   a) Therein said full-length DEN cDNA clone pRS/FLD2 produced in step 1(c) was linearized by BamHI digestion at position 1696 or 2203 of the DEN cDNA sequence (GenBank Accession No. AF038403) (There is a BamHI restriction site in DEN genome).
   b) A 96 nt DNA fragment was synthesized by fusion PCR containing 45 bases deriving from codon 6 to codon 20 of the structural protein at the 5' end, 45 bases deriving from codon 751 to codon 766 of the structural protein at the 3' end and the BamHI restriction site in the middle. The sequence of the DNA is provided as in SEQ ID NO:5.
   c) The linearized pRS/FLD2, 96 nt cDNA fragment and activated yeast were put into yeast transforming buffer(PEG buffer) and incubated at 30°C for one hour, then the linearized pRS/FLD2 and 96 nt cDNA fragment can be transformed into yeast automatically. After culture for two days, the transformed yeast colonies can be seen. Based on homologous recombination in yeast, the 96 nt cDNA fragment can replace structural protein sequences (SEQ ID NO:4) to produce DEN replicon cDNA clones with structural protein sequences deleted. Like recombinant HPV-DEN RNA replicons produced in Example 3 below, a DEN RNA replicon without structural protein sequences can be used to produce DEN RNA replicon virus-like partides (VLP) by at least four different packaging schemes as provided hereinabove, and will be described in Example 4 animal experiments below.

### Example 2

### Construction of HPV E7-E6-2A DNA Fragment

1. E7-E6 sequence is an HPV oncogene. (Gene Bank Accession No. AF486352; AF469197; and AF472508) designated as SEQ ID NO:1, which encodes an antigen containing 225 amino acids (SEQ ID NO:2).
2. Therein said 2A sequence is a mouth-foot-pestilence virus fragment containing sixty bases, designated as SEQ ID NO:3.
3. E6 and E7 fragments were produced by PCR with plasmid HPV-16 as template, oligos GCGAGAAATACGCCTTTCAATATGCTGAAACGCGAGAGAAACATGCATGGAGATACACCTA CA (SEQ ID NO: 12) and TGCAGTTCTCTTTTGGTGCATTGGTTTCTGAGAACAGATGGG (SEQ ID NO: 13) as one set of primers, oligos ATGCACCAAAAGAGAACTGCA (SEQ ID NO: 14) and AAGGTCAAAATTCAACAGCTGGGTTTCTCTACGTGT (SEQ ID NO: 15) as another set of primers. The 5' end of the E6 fragment contains the sequence, ATGCACCAAAAGAGAACTGCA which is identical to the 3' end of the E7 fragment. The 3' end of the E6 fragment contains the sequence, CAGCTGTTGAATTTTGACCTT which is identical to the 5' end of the 2A sequence. The 5' end of the E7 fragment contains a sequence identical to DEN II cDNA nt 108-124. A 111 bp DNA fragment containing the 2A sequence and its 3' end sequence identical to DEN II cDNA nt 2275-2375 was produced by fusion PCR with oligos CAGCTGTTGAATTTTGACCTTCTTAAGCTTGCGGGAGACGTCGAGTCCAACCCTGGCCCC (SEQ ID NO: 3) and ATACAGCGTCACGACTCCCACCAATACTAGTGACACAGACAGTGAGGTGCTGGGGCCAGG GTTGGAC TCGAC (SEQ ID NO: 16) as primers.
4. Full-length E7-E6-2A cDNA fragment was produced by PCR with the E6 fragment, the E7 fragment and the 111 bp fragment containing the 2A sequence as template, oligos GCGAGAAATACGCCTTTCAATATGCTGAAACGCGAGAGAAACATGCATGGAGATACACCTA CA (SEQ ID NO:17) and ATACAGCGTCACGACTCCCACCAATACTAGTGACACAGACAGTGAGGTGCTGGGGCCAGG GTTGGACTCGAC (SEQ ID NO:18) as primers. The 5' end and 3' end of the construct contains sequences identical to DEN cDNA gene.

### Example 3

### Integration of HPV Genome Into DEN RNA Replicon

1. Full-length DEN cDNA clone (pRS/FLD2) produced in step 1 (c) of Example 1 was linearized with BamHI.
2. The linearized DEN cDNA done (pRS/FLD2) and HPV E7-E6-2A cDNA fragment were transformed into yeast together. In the process of yeast DNA replication, the HPV-E7-E6-2A cDNA fragment can integrate into the DEN cDNA clone automatically and replace the DEN structural protein sequences (C, M and E coding region sequences) to produce a circular HPV-DEN RNA replicon cDNA clone (pRS/D2-HPV16).
3. The HPV-DEN RNA replicon cDNA clone produced in step 2 was purified with a Qiagen (Qiagen Inc.) column.
4. The HPV-DEN RNA replicon cDNA clone was transformed into Stab12™ (Invitrogen Inc., Carlsbad, CA) and a large number of HPV-DEN RNA replicon cDNA clones were produced by this system.
5. This HPV-DEN RNA replicon cDNA done was digested by Sacl at the 3' end.
6. The modified HPV-DEN RNA replicon cDNA was transcribed into RNA by DNA dependent Sp6 RNA polymerase to produce a recombinant HPV-DEN RNA replicon.
7. The recombinant HPV-DEN RNA replicon was transfected into BHK-21 (ATCC#CCL-10) cells by electroporation and recombinant HPV-DEN RNA replicons replicate and express E7-E6 protein of HPV.

### Example 4

### Four Ways of Production of HPV VLP

### Method 1: Production of VLP using tetracycline-regulated gene expression system

Summary: Using the tetracycline-regulated gene expression system, BHK-21 cells were transformed to produce a DEN structural protein regulated expression cell line. The Tet-Off Gene Expression systems were purchased from Clontech Inc. This plasmid expresses a regulatory protein named "rtTA" which regulates gene expression and plasmid transcription. This gene expression system contains pTet-Off regulatory vectors, pTRE2 response vectors and pTK-Hyg selection vectors.
1. DEN structural protein genes (full 2328 bp DEN structural protein genes designated as SEQ ID NO:4) were inserted into pTRE2 response vectors of the Tet-Off gene expression system. Namely the CpreME cDNA fragment was produced by PCR with full-length DEN cDNA clone (pRS/FLD2) as template, oligos ATATCCCCGCGGATGAATAACCAACGAA,4AAAGGCG (SEQ ID NO:19) and ATATATCTAGACTAGGCCTGCACCATAACTCCCAA (SEQ ID NO:20) as primers. The CpreME cDNA fragment and pTRE2 (Clontech Inc.) were digested by Xbal and Sac II. After Qiagen spin column (Qiagen Inc.) purification, these two fragments were ligated by T4 ligase (New England Biolab Inc.) and used to transform E. coli to produce recombinant pTRE2 containing DEN structural protein genes.
2. Transform BHK-21 cells with the pTet-Off regulatory vectors by electroporation and then BHK-21 Tet-Off cell lines are selected.
3. The Tet-Off gene expression system inserted with the structural protein genes produced in step 1 was transformed into BHK-21 Tet-Off cells by electroporation to produce a DEN structural proteins regulated-high expression BHK-21 cell line.
4. The recombinant HPV-DEN RNA replicon produced in Example 3 was transformed into said BHK-21 cell line by electroporation. After one day, the Tet-Off gene expression system was induced for high expression of structural proteins. After ten days, the supematant was collected to produce 10⁴ packaged recombinant HPV-DEN RNA replicon VLP per ml.

### Method 2: Production of HPV recombinants using Sindbis RNA replicon

1. Production of DEN structural protein fragment:
   a) A DNA fragment of a C protein gene was produced by PCR with pRS/FLD2 produced in Step 1 (c) of Example 1 as template, and oligos CCTCTAGCTAGAGCTTACCATGAATAACCAACGAAAAAAG (SEQ ID NO:21) and GATTAGAGCTCTTATCTGCGTCTCCTGTTCAAGAT (SEQ ID NO:22) as primers.
   b) A DNA fragment of prM-E gene was produced by PCR with pRS/FLD2 produced in Step 1(c) of Example 1 as template, and oligos GCGCTCTAGAATGACTGCAGGCATGATCATTATG (SEQ ID NO: 23) and ATGCCAGTAGGACAGGTGTAATCTAGGCCTGCACCATAACTCCCAA (SEQ ID NO: 24) as primers.
   c) A DNA fragment of Sindbis 26S was produced by PCR with Sindbis RNA replicon cDNA (Stratagene Co.) as template, and oligos ATTACACCTGTCCTACTGGCA (SEQ ID NO:25) and CATGGTAAGCTCTAGCTAGAG (SEQ ID NO:26) as primers.
   d) The 3' end of the prM-E DNA fragment and the 5' end of the Sindbis 26S DNA fragment contain an identical 21 nt DNA sequence. The 3' end of the Sindbis 26S DNA fragment and the 5' end of the C gene DNA fragment contain an identical 21 nt DNA sequence.
   e) The C/preM-E fragment and the Sindbis 26S DNA fragment were fused together by PCR with the three fragments as templates, and oligos GCGCTCTAGAATGACTGCAGGCATGATCATTATG (SEQ ID NO:27) and GTATAGAGCTCTTATCTGCGTCTCCTGTTCAAGAT (SEQ ID NO:28) as primers to produce DNA fragment preME-26S-C.
2. Production of recombinant Sindbis RNA replicon containing DEN structural protein genes.
   a) The PreME-26S-C fragment produced in step 1 and the Sindbis RNA replicon DNA plasmid (Stratagene Co.) were digested by Xbal and Sacl. After Qiagen spin column (Qiagen Inc.) purification, these two DNA fragments were ligated by T4 ligase (New England Biolab Inc.) and used to transform E. coli to produce recombinant Sindbis RNA replicon cDNA clones containing DEN structural protein genes and then was purified by Qiagen spin column (QIAGEN Inc.)
   b) Recombinant Sindbis RNA replicon cDNA clone produced in step (a) was linearized by Xhol.
   c) Linearized Sindbis RNA replicon cDNA clone was transcribed into RNA by DNA dependent Sp6 RNA polymerase to produce a recombinant Sindbis RNA replicon containing DEN structural protein genes.
3. Recombinant HPV-DEN RNA replicon produced in Example 3 was transfected into BHK-21 cells. After 24 hours, recombinant Sindbis RNA replicons produced in said step 2(c) were transfected into the cells again. The two transfections were done by electroporation with a 0.4 cm Gene Pulser Cuvette, 200 V/950 uF. After one or two days, the supematant was collected to produce 10⁴-10⁵ recombinant HPV- DEN RNA replicon VLP per ml.

### Method 3: Production of particles using Sindbis RNA replicon VLP.

1. Recombinant Sindbis RNA replicons produced in step 2(c) of Method 2 and DH-BB RNA (Stratagene Inc.) were transfected into BHK-21 cells (ATCC#CCL-10) together by electroporation. After three to five days, the supernatant was collected to obtain recombinant Sindbis RNA replicon VLP containing DEN structural protein genes.
2. Recombinant HPV-DEN RNA replicon VLPs produced in step 3 of Method 2 were infected into BHK-21 cells (ATCC#CCL-10). After 24 hours, recombinant Sindbis RNA replicon VLPs produced in said step 1 were infected into the cells again. After 24 to 48 hours, the supernatant was collected to obtain 10⁵-10⁶ recombinant HPV-DEN RNA replicon VLPs per ml.

### Method 4: Production of BHK-21 Packaging Cell line transformed with DEN cDNA sequence deleted NS3 region

1. A DNA fragment of cytomegalovirus (CMV) containing the immediate-early enhancer/promoter region was produced by PCR with pCl (Promega Co.) as template, oligos 5' CMV and 3' CMV as primers. As an early enhancer and promoter, this DNA fragment (SEQ ID NO:29) of CMV is 631 bp in length. The DNA fragment of the 5' end of DEN cDNA (DEN 5' end) was produced by PCR with pRS/FLD2 produced in Step 1(c) of Example 1 as template, and oligos 5' DEN 5' end and 3' DEN 5' end as primers. These two DNA fragments were fused together by fusion PCR with these two fragments as template, and oligos 5' CMV and 3' DEN 5' end as primers to produce the DNA fragment, CMV-DEN 5' end (CMV-DEN 5' end).
2. The CMV-DEN 5' end fragment produced in Step 1 and plasmid pRS424(ATCC#77105) were digested by Kpnl and Apal. After Qiagen spin column (Qiagen Inc.) purification, these two DNA fragments were ligated by T4 ligase (New England Biolab Inc.) and used to transform E. coli to produce CMV-DEN 5' end clone(pRS/CMV-DEN 5' end).
3. The DNA fragment of the 3' end of DEN cDNA (DEN 3' end) was produced by PCR with pRS/FLD2 produced in Step 1(c) of Example 1 as template, and oligo 5' DEN 3' end and 3' DEN 3' end as primers. A DNA fragment of the hepatitis delta virus antigenomic ribozyme (HDVr, SEQ ID NO:30) was produced by PCR with oligo 5' HDVr and 3' HDVr as primers. A DNA fragment of bovine growth hormone poly A (BGH pA) was produced by PCR with pcDNA3 (Invitrogen, Co.) as template, and oligo 5' pA and 3' pA as primers. The three DNA fragments were fused together by fusion PCR with the three fragments as templates, and oligos 5' DEN 3' end and 3' pA as primers to produce DNA fragment, DEN 3' end-HDVr-pA. 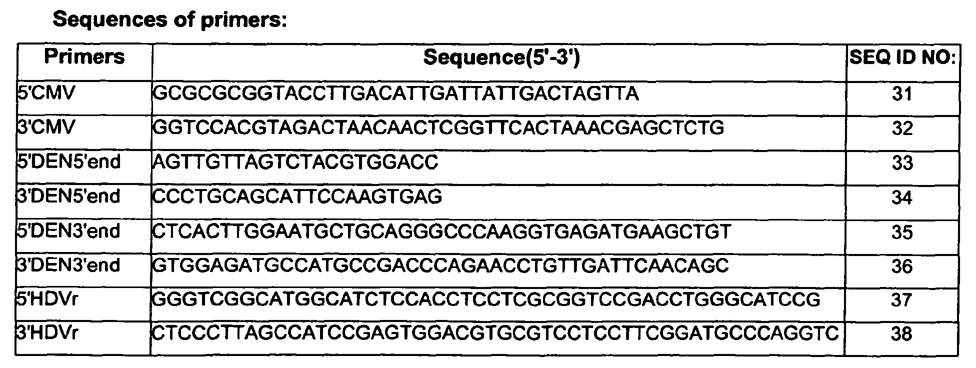 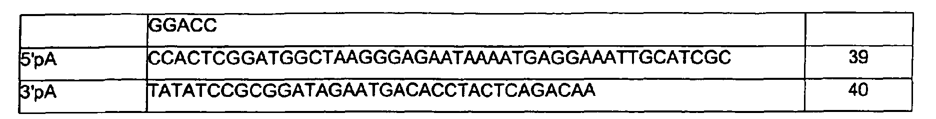
4. Plasmid pRS/CMV-DEN 5' end produced in step 2 and DNA fragment DEN 3' end- HDVr -pA produced in step 3 were digested by Apal and Sacil. After Qiagen spin column (Qiagen Inc.) purification, these two DNA fragments were ligated by T4 ligase (New England Biolab Inc.), transformed into E. coli and selected to produce pRS/CMV-DEN 5' end-DEN 3' end-HDVr-pA plasmid DNA.
5. Plasmid DNA pRS/CMV-DEN 5' end-DEN 3' end-HDVr-pA produced in step 4 was digested by Apal and plasmid pRS/FLD2 produced in step 1 (c) of Example 1 was digested by Xbal and Sacl. The two digestive products were purified, used to transform yeast and selected to produce full-length DEN RNA replicon cDNA clone (pRS/CMV/D2) according to homologous recombination in yeast.
6. Full-length DEN RNA replicon cDNA clone (pRS/CMV/D2) produced in step 5 was digested by Xhol to produce linearized full-length DEN RNA replicon cDNA clone. A 75 bp DNA fragment (CAGACTGAAAAAAGTATTGAAGACAATCCAGAGATCGAAGGAATTAAGAACAACCAAATCT TGGAAAATGTGGAG, SEQ ID NO:43) was produced by PCR with oligos CAGACTGAAAAAAGTATTGAAGACAATCCAGAGATCGAAGGAATTAAGAACAACCAAATC (SEQ ID NO:41) and CTCCACATTTTCCAAGATTTGGTTGTTCTTAATTCC (SEQ ID NO:42) as primers. The linearized DEN RNA replicon cDNA clone and 75 bp DNA fragment were used to transform yeast resulting in a construct where a part of the NS3 gene (nt 5059-6215) was deleted in the linearized DEN RNA replicon cDNA by homologous recombination in yeast.
7. The NS3- deleted DEN cDNA clone produced in step 6 and pcDNA3 (Invitrogen, Inc.) were transfected into BHK-21 cells (ATCC#CCL-10) together. After culture for one day, these cells were changed to culture medium containing G418 (Sigma). A stable G418-resistant packaging cell line was selected by transfection of recombinant HPV-DEN RNA replicon produced in Example 3 into G418-resistant cells. The titer of recombinant HPV-DEN RNA replicon VLP can reach 10⁶.

### Example 5

### Animal experiments of HPV Vaccine

To examine the immunity of a HPV expressing VLP, the HPV VLP was used to determine any effect on tumor cells using C57BU6 mice.
C57BU6 mice were inoculated with JHU-1 HPV cells as the tumor model, JHU-1 HPV cells contained E6 and E7 oncogenes of HPV. Eight-week-old mice were inoculated in the flank with 500 ul PBS buffer containing 10⁵ JHU-1 HPV cells. JHU-1 HPV cells can completely induce solid tumors. Seven days after inoculation of JHU-1 HPV cells, tumors could be felt. After fourteen days, the tumors could reach over 6 mm in diameter.

| **Rx of HPV VLP: Four groups of eight C57BL/6 mice** | | | | |
|---|---|---|---|---|
| | **flank inoculation day 1** | **day 14** | **Day 21** | **day 35** |
| group 1 control | | | | HPV E6/E7-specifi CD⁸⁺T cell assay |
| group 2 tumor control | 1X10⁵ JHU-1 Cells | 10⁷ PFU VLP | 10⁷ PFU VLP | |
| group 3 VLP treatment | 1X10⁵ JHU-1 Cells | 10⁷ PFU HPV-VPL | 10⁷ PFU HPV-VPL | |
| group 4 VLP control | 500 ul PBS | 10⁷ PFU HPV-VPL | 10⁷ PFU HPV-VPL | HPV E6/E7-specific CD⁸⁺ T cell assay |
| PFU = Infectious Partide of HPV replicon-containing VLP | | | | |

Groups 2 and 3 of eight mice each were inoculated in the flank with 10⁵ JHU-1 HPV cells. After fourteen days, groups 3 and 4 of eight mice each were inoculated ip with 10⁷ PFU HPV-VLP and after seven days they were inoculated with 10⁷ PFU HPV-VLP again.
1. To examine immunity of HPV VLP:
a) After inoculation of mice with JHU-1 HPV cells, tumor growth and sizes were examined every two days.
b) An HPV E6/E7-specific CD⁸⁺ T cell assay was used to examine the cells by cytoplasm staining and flow cytometry. After the second inoculation of group 4 mice with HPV-VPL, spleen cells were separated and used for staining with fluorescence-IFN-γ and TNF-α antibodies. Stained cell surface IFN and TNF can be detected with flow cytometry.

2. Results:
a) The effects of HPV VPL on tumor growth

| Group 2 (tumor control) | | | | | | | | |
|---|---|---|---|---|---|---|---|---|
| flank inoculation of JHU-1 Cells (day) | mice 1 tumor (mm) | mice 2 tumor (mm) | mice 3 tumor (mm) | mice 4 tumor (mm) | mice 5 tumor (mm) | mice 6 tumor (mm) | mice 7 tumor (mm) | mice 8 tumor (mm) |
| day14 | 6 | 6 | 7 | 7 | 7 | 8 | 8 | 8 |
| day21 | 12 | 14 | 15 | 15 | 16 | 16 | 17 | 17 |
| day28 | 15 | 16 | 17 | 18 | 18 | 18 | 19 | 20 |
| day35 | 19 | 22 | >25* | >25 | >25 | >25 | >25 | >25 |
| If a tumor is larger than 25mm, the mouse was sacrificed. | | | | | | | | |

| Group 3 (VPL treatment) | | | | | | | | |
|---|---|---|---|---|---|---|---|---|
| flank inoculation of JHU-1 Cells (day) | mice 1 tumor (mm) | mice 2 tumor (mm) | mice 3 tumor (mm) | mice 4 tumor (mm) | mice 5 tumor (mm) | mice 6 tumor (mm) | mice 7 tumor (mm) | mice 8 tumor (mm) |
| day14 | 6 | 7 | 7 | 7 | 7 | 8 | 8 | 8 |
| day21 | 3 | 5 | 5 | 6 | 6 | 7 | 9 | 10 |
| day28 | 0 | 0 | 0 | 0 | 2 | 4 | 8 | 10 |
| day35 | 0 | 0 | 0 | 0 | 0 | 4 | 9 | 11 |

In group 3, after inoculation of C57BU6 mice with 10⁵ JHU-HPV for fourteen days, tumors were induced, as expected. The tumor bumps sometimes were larger than 6 mm in diameter. Then those mice were inoculated ip with 10⁷ HPV-VPL. After two days it can be seen that most tumors began to shrink. After first inoculation, after seven days, the mice were inoculated with 10⁷ HPV-VPL again. After two inoculations for one week, tumors in almost half the mice disappeared. In other mice, the trend was for diminution of tumor size. In group 2, after inoculation of C57BU6 mice with 10⁵ JHU-HPV for thirty-five days, tumors of 75% of the mice were larger than 25mm.
b) The effects of HPV VLP on E6-E7-specific CD⁸⁺ T cells

| | **IFN-γ** | **TNF-α** |
|---|---|---|
| group 1 control | 0.04% | 0.08% |
| group 4 VPL control | 1.5% | 1.28% |

The effects of the HPV VPL on E6-E7-specific CD⁸⁺ T cells were examined by cytoplasm staining. The HPV VPL induced CD⁸⁺ T cells to secrete IFN-γ and TNF-α.

### Example 6

### Construction of Different Types of DEN II VPL Vectors

A recombinant DEN II replicon was constructed as in Examples 1, 2 and 3 as described, but the difference was that the sequence of the 5' end of the C gene and the length of the NS1 signal peptide were different. VLP were produced as in Example 4 described above.

| Results: | | | | |
|---|---|---|---|---|
| **retained C gene fragment** | **retained E gene fragment** | **inserted sequence** | **replication and expression** | **packaging** |
| 30 | 72 | no | no | no |
| 60 | 45 | HPV16 E7-E6-2A | no | no |
| 120 | 72 | HPV16 E7-E6-2A | yes | yes |
| 15 | 30 | HPV16 E7-E6-2A | no | no |
| 90 | 120 | HPV16 E7-E6-2A | yes | yes |
| 90 | 150 | HPV16 E7-E6-2A | yes | yes |
| 120 | 72 | HPV16 E7-E6 | no | no |

### Example 7

### Construction of DEN I VPL

A recombinant DEN replicon was constructed as in Examples 1, 2 and 3 above, but the difference is that DEN I replaced DEN II of Examples 1, 2 and 3. Then VLP were produced by method 4 of Example 4. Four mice of Example 5 with tumors were inoculated as group 3 of Example 5 described above.

All publications are herein incorporated by reference to the same extent as if each individual publication was specifically and individually indicated to be incorporated by reference in its entirety. It will occur to those of ordinary skill in the art that various modifications may be made 
Table of DNA sequences

<110> Shanghai Tengen Biomedical Co., LTD. Shanghai, China
Tengen Biomedical Co. Rockville, Maryland, USA.
Beijing Oriental Tengen Technology Development Co., LTD., Beijing, China

<120> Virus-like particle containing a dengue virus recombinant replicon

<130> S 10027 EP

<150> CN 03115273.2
<151> 2003-01-30

<150> CN 03115272.4
<151> 2003-01-30

<160> 44

<170> PatentIn version 3.1

<210> 1
<211> 768
<212> DNA
<213> Human papilloma virus

<400> 1
atgcatggag atacacctac attgcatgaa tatatgttag atttgcaacc agagacaact 60
gatctctact gttatgagca attaaatgac agctcagagg aggaggatga aatagatggt 120
ccagctggac aagcagaacc ggacagagcc cattacaata ttgtaacctt ctgttgcaag 180
tgtgactcta cgcttcggct gtgcgtacaa agcacacacg tagacattcg tacgttggaa 240
gacctgttaa tgggcacact aggaattgtg tgccccatct gttctcagaa accaatgcac 300
caaaagagaa ctgcaatgtt tcaggaccca caggagcgac ccagaaagtt accacagtta 360
tgcacagagc tgcaaacaac tatacatgat ataatattgg aatgtgtgta ctgcaagcaa 420
cagttactgc gacgtgaggt atatgacttt gcttttcggg atttatgcat agtatataga 480
gatgggaatc catatgctgt atgtgataaa tgtttaaagt tttattctaa aattagtgag 540
tatagacatt attgttatag tttgtatgga acaacattag aacagcaata caacaaaccg 600
ttgtgtgatt tgttaattag gtgtattaac tgtcaaaagc cactgtgtcc tgaagaaaag 660
caaagacatc tggacaaaaa gcaaagattc cataatataa ggggtcggtg gaccggtcga 720
tgtatgtctt gttgcagatc atcaagaaca cgtagagaaa cccagctg 768

<210> 2
<211> 256
<212> PRT
<213> Human papilloma virus

<400> 2
Met His Gly Asp Thr Pro Thr Leu His Glu Tyr Met Leu Asp Leu Gln
1               5                   10                  15
Pro Glu Thr Thr Asp Leu Tyr Cys Tyr Glu Gln Leu Asn Asp Ser Ser
            20                  25                  30
Glu Glu Glu Asp Glu Ile Asp Gly Pro Ala Gly Gln Ala Glu Pro Asp
        35                  40                  45
Arg Ala His Tyr Asn Ile Val Thr Phe Cys Cys Lys Cys Asp Ser Thr
    50                  55                  60
Leu Arg Leu Cys Val Gln Ser Thr His Val Asp Ile Arg Thr Leu Glu
65                  70                  75                  80
Asp Leu Leu Met Gly Thr Leu Gly Ile Val Cys Pro Ile Cys Ser Gln
                85                  90                  95
Lys Pro Met His Gln Lys Arg Thr Ala Met Phe Gln Asp Pro Gln Glu
            100                 105                 110
 
Arg Pro Arg Lys Leu Pro Gln Leu Cys Thr Glu Leu Gln Thr Thr Ile
        115                 120                 125
His Asp Ile Ile Leu Glu Cys Val Tyr Cys Lys Gln Gln Leu Leu Arg
    130                 135                 140
Arg Glu Val Tyr Asp Phe Ala Phe Arg Asp Leu Cys Ile Val Tyr Arg
145                 150                 155                 160
Asp Gly Asn Pro Tyr Ala Val Cys Asp Lys Cys Leu Lys Phe Tyr Ser
                165                 170                 175
Lys Ile Ser Glu Tyr Arg His Tyr Cys Tyr Ser Leu Tyr Gly Thr Thr
            180                 185                 190
Leu Glu Gln Gln Tyr Asn Lys Pro Leu Cys Asp Leu Leu Ile Arg Cys
        195                 200                 205
Ile Asn Cys Gln Lys Pro Leu Cys Pro Glu Glu Lys Gln Arg His Leu
    210                 215                 220
Asp Lys Lys Gln Arg Phe His Asn Ile Arg Gly Arg Trp Thr Gly Arg
225                 230                 235                 240
Cys Met Ser Cys Cys Arg Ser Ser Arg Thr Arg Arg Glu Thr Gln Leu
                245                 250                 255

<210> 3
<211> 60
<212> DNA
<213> foot-and-mouth disease virus

<400> 3
cagctgttga attttgacct tcttaagctt gcgggagacg tcgagtccaa ccctggcccc      60

<210> 4
<211> 2328
<212> DNA
<213> Dengue virus

<400> 4
atgaataacc aacgaaaaaa ggcgagaaat acccctttca atatgctgaa acgcgagaga      60
aaccgcgtgt cgactgtaca acagctgaca aagagattct cacttggaat gctgcaggga     120
cgaggaccat taaaactgtt catggccctg gtggcgttcc ttcgtttcct aacaatccca     180
ccaacagcag ggatactgaa gagatgggga acaattaaaa aatcaaaagc cattaatgtt     240
ttgagagggt tcaggaaaga gattggaagg atgctgaaca tcttgaacag gagacgcaga     300
actgcaggca tgatcattat gctgattcca acagtgatgg cgttccattt aaccacacgt     360
aacggagaac cacacatgat cgtcagtaga caagagaaag ggaaaagtct tctgtttaaa     420
acagaggatg gtgtgaacat gtgtaccctc atggccatgg accttggtga attgtgtgaa     480
gatacaatca cgtacaagtg tccttttctc aggcagaatg aaccagaaga catagattgt     540
tggtgcaact ctacgtccac atgggtaact tatgggacgt gtaccaccac aggagaacac     600
agaagagaaa aaagatcagt ggcactcgtt ccacatgtgg gaatgggact ggagacacga     660
actgaaacat ggatgtcatc agaaggggcc tggaaacatg cccagagaat tgaaacttgg     720
atcttgagac atccaggctt taccataatg gcagcaatcc tggcatacac cataggaacg     780
acacatttcc aaagagccct gattttcatc ttactgacag ctgtcgctcc ttcaatgaca     840
atgcgttgca taggaatatc aaatagagac tttgtagaag gggtttcagg aggaagctgg     900
gttgacatag tcttagaaca tggaagctgt gtgacgacga tggcaaaaaa caaaccaaca     960
ttggattttg aactgataaa aacagaagcc aaacaacctg ccactctaag gaagtactgt    1020
atagaggcaa agctgaccaa cacaacaaca gattctcgct gcccaacaca aggagaaccc    1080
agcctaaatg aagagcagga caaaaggttc gtctgcaaac actccatggt ggacagagga    1140
tggggaaatg gatgtggatt atttggaaaa ggaggcattg tgacctgtgc tatgttcaca    1200
tgcaaaaaga acatgaaagg aaaagtcgtg caaccagaaa acttggaata caccattgtg    1260
ataacacctc actcagggga agagcatgca gtcggaaatg acacaggaaa acatggcaag    1320
gaaatcaaaa taacaccaca gagttccatc acagaagcag agttgacagg ctatggcact    1380
gtcacgatgg agtgctctcc gagaacgggc ctcgacttca atgagatggt gttgctgcaa    1440
atggaaaata aagcttggct ggtgcacagg caatggttcc tagacctgcc gttgccatgg    1500
ctgcccggag cggacacaca aggatcaaat tggatacaga aagagacatt ggtcactttc    1560
aaaaatcccc atgcgaagaa acaggatgtt gttgttttgg gatcccaaga aggggccatg    1620
 
cacacagcac tcacaggggc cacagaaatc cagatgtcat caggaaactt actgttcaca 1680
ggacatctca agtgcaggct gaggatggac aaactacagc tcaaaggaat gtcatactct 1740
atgtgcacag gaaagtttaa agttgtgaag gaaatagcag aaacacaaca tggaacaata 1800
gttatcagag tacaatatga aggggacggt tctccatgta agatcccttt tgagataatg 1860
gatttggaaa aaagacatgt tttaggtcgc ctgattacag tcaacccaat cgtaacagaa 1920
aaagatagcc cagtcaacat agaagcagaa cctccattcg gagacagcta catcatcata 1980
ggagtagagc cgggacaatt gaagctcaac tggtttaaga aaggaagttc tatcggccaa 2040
atgattgaga caacaatgag gggagcgaag agaatggcca ttttaggtga cacagcttgg 2100
gattttggat ccctgggagg agtgtttaca tctataggaa aggctctcca ccaagttttc 2160
ggagcaatct atggggctgc cttcagtggg gtctcatgga ctatgaaaat actcatagga 2220
gtcattatca catggatagg aatgaattca cgcagcacct cactgtctgt gtcactagta 2280
ttggtgggag tcgtgacgct gtatttggga gttatggtgc aggcctga 2328

<210> 5
<211> 96
<212> DNA
<213> Artificial sequences

<400> 5
aaaaaggcga gaaatacgcc tttcaatatg ctgaaacgcg agagaggatc cagcacctca 60
ctgtctgtgt cactagtatt ggtgggagtc gtgacg 96

<210> 6
<211> 56
<212> DNA
<213> Artificial sequences
<400> 6
tgcacattcg ctctagaatt taggtgacac tatagagttg ttagtctacg tggacc 56

<210> 7
<211> 18
<212> DNA
<213> Artificial sequences
<400> 7
gcttcccgga ggagtggc 18

<210> 8
<211> 18
<212> DNA
<213> Artificial sequences
<400> 8
gacccagcaa gtatagcg 18

<210> 9
<211> 21
<212> DNA
<213> Artificial sequences

<400> 9
gacatgggga tgggttcttc a 21

<210> 10
<211> 39
<212> DNA
<213> Artificial sequences

<400> 10
acatgcttcg acctcgaggt ggacctcggt tgcggcaga 39

<210> 11
<211> 58
 
<212> DNA
<213> Artificial sequences
<400> 11
ctggaatcag ctgagctcag aacctgttga ttcaacagca ccattccatt ttctggcg 58

<210> 12
<211> 63
<212> DNA
<213> Artificial sequences
<400> 12
gcgagaaata cgcctttcaa tatgctgaaa cgcgagagaa acatgcatgg agatacacct 60
aca 63

<210> 13
<211> 42
<212> DNA
<213> Artificial sequences
<400> 13
tgcagttctc ttttggtgca ttggtttctg agaacagatg gg 42

<210> 14
<211> 21
<212> DNA
<213> Artificial sequences
<400> 14
atgcaccaaa agagaactgc a 21

<210> 15
<211> 36
<212> DNA
<213> Artificial sequences
<400> 15
aaggtcaaaa ttcaacagct gggtttctct acgtgt 36

<210> 16
<211> 72
<212> DNA
<213> Artificial sequences
<400> 16
atacagcgtc acgactccca ccaatactag tgacacagac agtgaggtgc tggggccagg 60
gttggactcg ac 72

<210> 17
<211> 63
<212> DNA
<213> Artificial sequences
<400> 17
gcgagaaata cgcctttcaa tatgctgaaa cgcgagagaa acatgcatgg agatacacct 60
aca 63

<210> 18
<211> 72
<212> DNA
<213> Artificial sequences
<400> 18
atacagcgtc acgactccca ccaatactag tgacacagac agtgaggtgc tggggccagg 60
gttggactcg ac 72

<210> 19
<211> 36
<212> DNA
 
<213>  Artificial sequences
<400>  19
atatccccgc ggatgaataa ccaacgaaaa aaggcg                                36

<210>  20
<211>  35
<212>  DNA
<213>  Artificial sequences
<400>  20
atatatctag actaggcctg caccataact cccaa                                 35

<210>  21
<211>  40
<212>  DNA
<213>  Artificial sequences
<400>  21
cctctagcta gagcttacca tgaataacca acgaaaaaag                            40

<210>  22
<211>  35
<212>  DNA
<213>  Artificial sequences
<400>  22
gattagagct cttatctgcg tctcctgttc aagat                                 35

<210>  23
<211>  34
<212>  DNA
<213>  Artificial sequences
<400>  23
gcgctctaga atgactgcag gcatgatcat tatg                                  34

<210>  24
<211>  46
<212>  DNA
<213>  Artificial sequences
<400>  24
atgccagtag gacaggtgta atctaggcct gcaccataac tcccaa                     46

<210>  25
<211>  21
<212>  DNA
<213>  Artificial sequences
<400>  25
attacacctg tcctactggc a                                                21

<210>  26
<211>  21
<212>  DNA
<213>  Artificial sequences
<400>  26
catggtaagc tctagctaga g                                                21

<210>  27
<211>  34
<212>  DNA
<213>  Artificial sequences
<400>  27
gcgctctaga atgactgcag gcatgatcat tatg                                  34

<210>  28
 
<211> 35
<212> DNA
<213> Artificial sequences
<400> 28
gtatagagct cttatctgcg tctcctgttc aagat 35

<210> 29
<211> 595
<212> DNA
<213> cytomegalo virus

<400> 29
cattgattat tgactagtta ttaatagtaa tcaattacgg ggtcattagt tcatagccca 60
tatatggagt tccgcgttac ataacttacg gtaaatggcc cgcctggctg accgcccaac 120
gacccccgcc cattgacgtc aataatgacg tatgttccca tagtaacgcc aatagggact 180
ttccattgac gtcaatgggt ggagtattta cggtaaactg cccacttggc agtacatcaa 240
gtgtatcata tgccaagtcc gccccctatt gacgtcaatg acggtaaatg gcccgcctgg 300
cattatgccc agtacatgac cttacgggac tttcctactt ggcagtacat ctacgtatta 360
gtcatcgcta ttaccatggt gatgcggttt tggcagtaca ccaatgggcg tggatagcgg 420
tttgactcac ggggatttcc aagtctccac cccattgacg tcaatgggag tttgttttgg 480
caccaaaatc aacgggactt tccaaaatgt cgtaataacc ccgccccgtt gacgcaaatg 540
ggcggtaggc gtgtacggtg ggaggtctat ataagcagag ctcgtttagt gaacc 595

<210> 30
<211> 84
<212> DNA
<213> Hepatitis virus

<400> 30
gggtcggcat ggcatctcca cctcctcgcg gtccgacctg ggcatccgaa ggaggacgca 60
cgtccactcg gatggctaag ggag 84

<210> 31
<211> 36
<212> DNA
<213> Artificial sequences
<400> 31
gcgcgcggta ccttgacatt gattattgac tagtta 36

<210> 32
<211> 42
<212> DNA
<213> Artificial sequences
<400> 32
ggtccacgta gactaacaac tcggttcact aaacgagctc tg 42

<210> 33
<211> 21
<212> DNA
<213> Artificial sequences
<400> 33
agttgttagt ctacgtggac c 21

<210> 34
<211> 21
<212> DNA
<213> Artificial sequences
<400> 34
ccctgcagca ttccaagtga g 21

<210> 35
 
<211> 42
<212> DNA
<213> Artificial sequences
<400> 35
ctcacttgga atgctgcagg gcccaaggtg agatgaagct gt 42

<210> 36
<211> 42
<212> DNA
<213> Artificial sequences
<400> 36
gtggagatgc catgccgacc cagaacctgt tgattcaaca gc 42

<210> 37
<211> 48
<212> DNA
<213> Artificial sequences

<400> 37
gggtcggcat ggcatctcca cctcctcgcg gtccgacctg ggcatccg 48

<210> 38
<211> 55
<212> DNA
<213> Artificial sequences
<400> 38
ctcccttagc catccgagtg gacgtgcgtc ctccttcgga tgcccaggtc ggacc 55

<210> 39
<211> 45
<212> DNA
<213> Artificial sequences

<400> 39
ccactcggat ggctaaggga gaataaaatg aggaaattgc atcgc 45

<210> 40
<211> 35
<212> DNA
<213> Artificial sequences
<400> 40
tatatccgcg gatagaatga cacctactca gacaa 35

<210> 41
<211> 60
<212> DNA
<213> Artificial sequences
<400> 41
cagactgaaa aaagtattga agacaatcca gagatcgaag gaattaagaa caaccaaatc 60

<210> 42
<211> 36
<212> DNA
<213> Artificial sequences
<400> 42
ctccacattt tccaagattt ggttgttctt aattcc 36

<210> 43
<211> 75
<212> DNA
<213> Artificial sequences
 
<400> 43
cagactgaaa aaagtattga agacaatcca gagatcgaag gaattaagaa caaccaaatc 60
ttggaaaatg tggag 75

<210> 44
<211> 5
<212> PRT
<213> Artificial sequences
<220>
<221> misc_feature
<222> (1).(5)
<223> substrate of signal peptide protease

<400> 44
Pro Gln Ala Gln Ala
1               5
 to the disclosed embodiments and that such modifications are intended to be within the scope of the present invention, which is defined by the following claims.

## Claims

1. A recombinant DEN replicon with a deletion of preM and comprising of follow nucleotide sequences at least:
a) DEN 5' untranslated region (5'-UTR);
b) The coding region of the first 20 amino acids of the DEN capsid protein;
c) The coding region of the signal peptide of the DEN NS1 protein;
d) The coding region of all the DEN non-structure proteins;
e) DEN 3'-end untranslation region (3'-LTTR);
f) Exogenous nucleotide sequences such as a tumor antigen gene, a virus antigen gene, an immunoregulator gene and so on locate the region between b) and c).

2. The particle of claim 1, wherein said the DEN replicon that the 3' end of any exogenous nucleotide sequence contains 3' release elements upstream of the coding region of the NS1 signal peptide, which can be the 5' release elements. These release elements can be a nucleotide sequence obtained from the autoprotease of foot-and-mouth disease virus (FMDV) (SEQ ID NO: 3), or nucleotide sequence encoding the substrate of signal peptide protease (SEQ ID NO: 44) or both.

3. The particle of claim 1, wherein said the DEN replicon is obtained from any one of the following: DEN type I, DEN type II, DEN type III, or DEN type IV.

4. The particle of claim 1, wherein said the DEN replicon includes a 100-2,000bp of exogenous nucleotide sequences;

5. A virus-like particles comprising the particle of claim 1 and DEN structural proteins.

6. A pharmaceutical compositions containing the particle of claim 1 or a DEN-VLP, and pharmaceutically acceptable carrier.

7. The particle of claim 1, wherein said the usage of DEN replicon is as a pro-drug for prophylactic and therapeutic purposes to cancer and viral infection.

8. The particle of claim 1, wherein said the usage of DEN replicon is through a packaging system encapsulating DEN recombinant replicons into VLP. These packaging cells are selected from:
a) Cells transfected by a plasmid with deleted structural genes of a Dengue recombinant replicon;
b) Cells transfected by a vector derived from a helper virus containing sequences of the structural proteins which are deleted from the DEN recombinant replicon (a);
c) Cells integrated with structural genes which are deleted from the DEN recombinant replicon (a) in the genome thereof, and above cells express DEN structural proteins for complementing replicon packaging and the structural gene expression doesn't affect the growth of the host cells.

9. A method of making a virus-like particle, comprising the steps of:
a) Introducing into a DEN packaging cell line, a DEN recombinant replicon or a VLP containing said replicon of claim 1;
b) Culturing that cell line either so it can express the DEN structural proteins; or introducing into that cell line replicons of a helper virus containing a DEN structural gene if the cell line cannot express that DEN structural gene, then culturing the cell line; and
c) Collecting DEN-VLP containing DEN recombinant replicons.

10. The method of claim 9, wherein said packaging cells contain a DEN structural protein expression vector, selected from a vector with an NS3 deletion and a Tet-regulated vector.
